(19) Europäisches Patentamt
European Patent Office
Office européen des brevets

(11) Veröffentlichungsnummer : **0 416 338 B1**

(12) # EUROPÄISCHE PATENTSCHRIFT

(45) Veröffentlichungstag der Patentschrift :
**14.07.93 Patentblatt 93/28**

(51) Int. Cl.[5] : **C08G 18/79,** C08G 18/80, C07D 251/34

(21) Anmeldenummer : **90115655.4**

(22) Anmeldetag : **16.08.90**

(54) **Verfahren zur Herstellung von Lösungen von Isocyanuratgruppen aufweisenden Polyisocyanaten in Lacklösungsmitteln und ihre Verwendung.**

(30) Priorität : **29.08.89 DE 3928503**

(43) Veröffentlichungstag der Anmeldung :
**13.03.91 Patentblatt 91/11**

(45) Bekanntmachung des Hinweises auf die Patenterteilung :
**14.07.93 Patentblatt 93/28**

(84) Benannte Vertragsstaaten :
**AT BE CH DE ES FR GB GR IT LI NL SE**

(56) Entgegenhaltungen :
**DE-A- 2 737 211**

(56) Entgegenhaltungen :
**FR-A- 1 455 546**
**FR-A- 2 235 147**
**GB-A- 840 318**
**US-A- 4 491 663**
**US-A- 4 607 103**

(73) Patentinhaber : **BAYER AG**
**W-5090 Leverkusen 1 Bayerwerk (DE)**

(72) Erfinder : **Pedain, Josef, Dr.**
**Haferkamp 6**
**W-5000 Köln 80 (DE)**
Erfinder : **Margotte, Dieter, Dr.**
**Wedelstrasse 48**
**W-4150 Krefeld 1 (DE)**

EP 0 416 338 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von Lösungen einer verbesserten Aromatenverdünnbarkeit von Isocyanuratgruppen aufweisenden Polyisocyanaten auf Basis von Diisocyanatotoluolen in gegenüber Isocyanatgruppen inerten Lösungsmitteln und die Verwendung dieser Lösungen zur Herstellung von Zweikomponenten-Polyurethanlacken.

2,4- und 2,6-Diisocyanatotoluol und vor allem die technischen Gemische dieser beiden Diisocyanate sind wichtige industrielle Rohstoffe, die auch auf dem Gebiet der Lackierungen und Beschichtungen eine bedeutende Rolle spielen. Als Lackrohstoffe finden sie u.a. Verwendung in Form von Urethan- oder Isocyanuratgruppen aufweisenden Derivaten.

Insbesondere die Isocyanuratgruppen aufweisenden Polyisocyanate auf Basis von 2,4- und gegebenenfalls 2,6-Diisocyanatotoluol stellen wertvolle Bestandteile für Zweikomponenten-Polyurethanlacke für die Holz- und Möbellackierung dar. Die Herstellung der modifizierten Polyisocyanate geschieht im allgemeinen durch Teiltrimerisierung der Isocyanatgruppen von 2,4- und gegebenenfalls 2,6-Diisocyanatotoluol in 30- bis 70-gew.-%iger Lösung in geeigneten Lacklösungsmitteln (vgl. z.B. DE-OS 2 414 413). Zur Herstellung von gebrauchsfertigen Lacken werden die dabei anfallenden Lösungen oftmals mit Lösungen von Hydroxylgruppen aufweisenden, fettsäuremodifizierten Polyestern in Toluol, Xylol oder Gemischen dieser Lösungsmittel mit Benzinfraktionen kombiniert. Hierbei können jedoch wegen der beschränkten Verträglichkeit der Polyisocyanatlösungen mit schwach polaren Lösungsmitteln der genannten Art oder mit den in den Lösungsmitteln gelösten, Hydroxylgruppen aufweisenden Bindemittelkomponenten leicht Unverträglichkeiten eintreten, die sich in Trübungen und Niederschlägen bemerkbar machen, was die Verwendbarkeit der Lösungen der Polyisocyanate sehr stark einschränkt.

Es war daher die der Erfindung zugrundeliegende Aufgabe ein neues Verfahren zur Herstellung von Lösungen von Isocyanuratgruppen aufweisenden Polyisocyanaten auf Basis von 2,4- und gegebenenfalls 2,6-Diisocyanatotoluol zur Verfügung zu stellen, welches zu Lösungen einer verbesserten Verdünnbarkeit mit schwach polaren Lösungsmitteln der genannten Art, insbesondere einer verbesserten Aromatenverdünnbarkeit führt.

Diese Aufgabe konnte dadurch gelöst werden, daß die als Ausgangsdiisocyanate eingesetzten Diisocyanatotoluole mit unterschüssigen Mengen bestimmter, gegebenenfalls Ethergruppen aufweisender Alkanole urethanisiert werden und die urethanisierten Ausgangsdiisocyanate anschließend in Form von 30- bis 70-gew.-%igen Lösungen in Lacklösungsmitteln einer Teiltrimerisierung unterzogen werden, bis der Gehalt der Lösungen an freiem Ausgangsdiisocyanat unter 0,5 Gew.-% abgesunken ist.

Es war bereits bekannt, 2,4- und gegebenenfalls 2,6-Diisocyanatotoluol durch Urethanisierung eines Teils der Isocyanatgruppen und Trimerisierung eines weiteren Teils der Isocyanatgruppen zu modifizieren, um hierdurch besondere Effekte zu erzielen.

So beschreibt beispielsweise die DE-OS 2 414 413 die Umsetzung von Lösungen von Isocyanuratgruppen aufweisenden Polyisocyanaten in Lacklösungsmitteln, die neben den Isocyanuratgruppen aufweisenden Polyisocyanaten noch erhebliche Mengen an freiem Ausgangsdiisocyanat enthalten, mit gegebenenfalls Ethergruppen aufweisenden Alkanolen, um durch eine weitgehend selektive Urethanisierung dieser freien Ausgangsdiisocyanate deren Gehalt auf unter 0,7 Gew.-%, bezogen auf Feststoff, zu erniedrigen. Der wesentliche Unterschied dieser Arbeitsweise gegenüber dem nachstehend näher beschriebenen erfindungsgemäßen Verfahren besteht somit in der Reihenfolge der Umsetzungen. Erfindungsgemäß wird zuerst urethanisiert und anschließend trimerisiert, während gemäß DE-OS 2 414 413 die Urethanisierung im Anschluß an die Trimerisierung erfolgt, um, wie gesagt, einen völlig andersartigen Effekt sicherzustellen.

Beim Verfahren der DE-OS 2 452 532 erfolgt die Teiltrimerisierung der Isocyanatgruppen von aromatischen Diisocyanaten, insbesondere von Diisocyanatotoluolen unter Verwendung eines binären Katalysatorsystems, bestehend aus Mannich-Basen und Carbamidsäureestern aus Isocyanaten und Alkoholen mit sekundären Hydroxylgruppen, wobei diese Carbamidsäureester beispielsweise in situ durch Urethanisierung eines Teils der Isocyanatgruppen des Ausgangsdiisocyanats mit bestimmten sekundären Alkoholen hergestellt werden können. In der Praxis läuft dies darauf hinaus, daß zuerst eine Teilurethanisierung und anschließend eine Teiltrimerisierung der Isocyanatgruppen vorgenommen wird. Die Reaktionsfolge entspricht somit der Reaktionsfolge des nachstehend näher beschriebenen erfindungsgemäßen Verfahrens. Unter den in der Vorveröffentlichung erwähnten sekundären Alkoholen finden sich auch Anlagerungsprodukte von Propylenoxid an höhere Alkanole wie beispielsweise 1-Decanol oder 1-Oktadecanol, jedoch handelt es sich bei diesen Alkoholen offensichtlich nicht um bevorzugte Ausgangsmaterialien, da in den Ausführungsbeispielen einfache sekundäre Alkohole wie beispielsweise Bis-(2-hydroxypropyl)-ether oder Anlagerungsprodukte von Propylenoxid an einfache mehrwertige Alkohole wie Propylenglykol oder Trimethylolpropan zur Anwendung gelangen. Andererseits gehören sekundäre Etheralkohole beim erfindungsgemäßen Verfahren keineswegs zu den bevorzugten

Alkoholen.

Der wesentliche Unterschied des nachstehend näher beschriebenen erfindungsgemäßen Verfahrens gegenüber dem Verfahren der DE-OS 2 452 532 ist jedoch in der völlig unterschiedlichen Aufgabenstellung und der unterschiedlichen Lösung der unterschiedlichen Aufgabe zu sehen. Die der Erfindung gemäß DE-OS 2 452 532 zugrundeliegende Aufgabe bestand nämlich darin, ein Verfahren zur Trimerisierung eines Teils der Isocyanatgruppen der Ausgangsdiisocyanate zur Verfügung zu stellen, welches nicht auf die Verwendung von Katalysatorengiften angewiesen ist und auf einfache Weise durch die Temperaturführung und die Menge der eingesetzten Katalysatoren steuerbar ist. Die Lösung dieser Aufgabe bestand in der Verwendung der genannten binären Katalysatorensysteme unter Trimerisierung der Ausgangsdiisocyanate in Substanz, um ungehindert von irgendwelchen Lösungsmitteln die Trimerisierung beim jeweils gewünschten Trimerisierungsgrad durch einen Hitzeschock abzubrechen. Es versteht sich, daß eine mit einem derartigen Verfahren befaßte Vorveröffentlichung in keinerlei näherem Zusammenhang mit der erfindungsgemäßen Aufgabe bzw. der erfindungsgemäßen Lösung dieser Aufgabe gebracht werden kann.

Gegenstand der Erfindung ist ein Verfahren zur Herstellung von Lösungen von Isocyanuratgruppen aufweisenden Polyisocyanaten in gegenüber Isocyanatgruppen inerten Lacklösungsmitteln durch Umsetzung von 2,5 bis 7 % der Isocyanatgruppen von 2,4-Diisocyanatotoluol oder von technischen Gemischen aus 2,4-Diisocyanatotoluol mit 2,6-Diisocyanatotoluol mit einem einwertigen Alkohol und anschließende Trimerisierung eines Teils der nach der Urethanisierung noch vorliegenden Isocyanatgruppen in Gegenwart eines die Trimerisierung von Isocyanatgruppen beschleunigenden Katalysators und Abbruch der Trimerisierungsreaktion durch Zugabe eines Katalysatorengifts, das dadurch gekennzeichnet ist, daß man

i) als einwertige Alkohole solche der allgemeinen Formel

$$R^1-[-O-CH_2-\underset{\displaystyle R^2}{\underset{|}{CH}}-]_n-OH$$

verwendet, wobei

- $R^1$ für einen gegebenenfalls olefinisch ungesättigten (cyclo)aliphatischen Kohlenwasserstoffrest mit 6 bis 18 Kohlenstoffatomen steht,
- $R^2$ für Wasserstoff oder eine Methylgruppe steht und
- n für 0 oder eine ganze Zahl von 1 bis 3 steht,

ii) die urethanisierten Diisocyanate der Trimerisierungsreaktion in Form von 30- bis 70-gew.-%igen Lösungen in gegenüber Isocyanatgruppen inerten Lacklösungsmitteln zuführt und

iii) die Trimerisierungsreaktion durch Zugabe eines Katalysatorengifts abbricht, nachdem der Gehalt des Reaktionsgemischs an freiem Diisocyanatotoluol unter 0,5 Gew.-% liegt.

Gegenstand der Erfindung ist auch die Verwendung der nach diesem Verfahren erhaltenen Lösungen von Isocyanuratgruppen aufweisenden Polyisocyanaten zur Herstellung von Zweikomponenten-Polyurethanlacken.

Ausgangsmaterialien für das erfindungsgemäße Verfahren sind 2,4-Diisocyanatotoluol oder dessen technischen Gemische mit 2,6-Diisocyanatotoluol. Vorzugsweise werden Gemische der genannten Isomeren im Gewichtsverhältnis 2,4:2,6=3:2 bis 9:1 eingesetzt. Besonders bevorzugt ist ein Gemisch, welches aus 2,4- und 2,6-Diisocyanatotoluol im Gewichtsverhältnis 3,9:1 bis 4.1:1 besteht.

Zumindest die zweite Stufe des erfindungsgemäßen Verfahrens erfolgt in Gegenwart von gegenüber Isocyanatgruppen inerten Lacklösungsmitteln. Derartige Lacklösungsmittel sind beispielsweise Ethylacetat, Butylacetat, Methylethylketon, Methylisobutylketon, Methoxypropylacetat oder Gemische derartiger Lösungsmittel.

Die erste Stufe des erfindungsgemäßen Verfahrens besteht in der Umsetzung von 2,5 bis 7 %, vorzugsweise 3 bis 5 % der Isocyanatgruppen der gelöst vorliegenden Ausgangsdiisocyanate mit einem einwertigen Alkohol oder einem Gemisch einwertiger Alkohole der nachstehend genannten Art.

Geeignete einwertige Alkohole sind solche der allgemeinen Formel

$$R^1-[-O-CH_2-\underset{\displaystyle R^2}{\underset{|}{CH}}-]_n-OH$$

wobei

$R^1$ für einen gegebenenfalls olefinisch ungesättigten (cyclo)aliphatischen Kohlenwasserstoffrest mit 6 bis 18 Kohlenstoffatomen, vorzugsweise einen gesättigten aliphatischen Kohlenwasserstoffrest mit 8 bis 12 Kohlenstoffatomen steht,

$R^2$ für Wasserstoff oder eine Methylgruppe, vorzugsweise für Wasserstoff steht und

n für 0 oder eine ganze Zahl von 1 bis 3, vorzugsweise 0 steht.

Beispiele für geeignete einwertige Alkohole sind 1-Hexanol, Cyclohexanol, 1-Octanol, 2-Ethyl-1-butanol, 2-Ethyl-1-hexanol, 4-Methyl-2-pentanol, 1-Decanol, 1-Dodecanol, 1-Tetradecanol, Stearylalkohol, 9-Oktadecen-1-ol, die den gemachten Ausführungen entsprechenden Ethoxilierungs- und/oder Propoxilierungsprodukte dieser Alkohole oder beliebige Gemische derartiger Alkohole. Besonders bevorzugt wird 2-Ethylhexanol-1 als einwertiger Alkohol verwendet.

Die erste Stufe des erfindungsgemäßen Verfahrens besteht in der Urethanisierung eines Teils der Isocyanatgruppen des Ausgangsdiisocyanats mit der genannten Alkoholkomponente, wobei 2,5 bis 7, vorzugsweise 3 bis 5 % der Isocyanatgruppen der Ausgangsdiisocyanate durch Umsetzung mit dem einwertigen Alkohol oder einem Gemisch aus verschiedenen einwertigen Alkoholen der beispielhaft genannten Art urethanisiert werden. Diese Modifizierung des Diisocyanats mit den Monoalkoholen erfolgt bei 0 bis 120°C, insbesondere bei 20 bis 80°C in Substanz oder in Lösung, wobei als Lösungsmittel solche der bereits oben beispielhaft genannten Art verwendet werden, die im Anschluß an die Urethanisierung als Reaktionsmedium für die Teiltrimerisierung dienen. Im Falle der Urethanisierung in Abwesenheit von Lösungsmitteln werden die teilurethanisierten Ausgangsdiisocyanate vor der Trimerisierung in einem Lösungsmittel oder Lösungsmittelgemisch der beispielhaft genannten Art gelöst.

Die anschließende Teiltrimerisierung der Isocyanatgruppen erfolgt auf katalytischem Wege bei 20 bis 80°C, wobei 30- bis 70-gew.-%ige Lösungen der anurethanisierten Ausgangsdiisocyanate zum Einsatz gelangen.

Als Trimerisierungskatalysatoren kommen grundsätzlich alle bekannten Trimerisierungskatalysatoren des Standes der Technik wie beispielsweise Phosphine, Alkalisalze, Alkalialkoholate, tert.-Amine u. dgl. in Betracht. Vorzugsweise werden jedoch Mannich-Basen der bereits in DE-OS 2 452 532 genannten Art als Trimerisierungskatalysatoren verwendet. Die Trimerisierung wird so lange fortgesetzt, bis der Gehalt des Reaktionsgemischs an Urethangruppen- und Isocyanuratgruppen-freiem Ausgangsdiisocyanat unter 0,5 Gew.-%, bezogen auf Lösung, abgesunken ist. Dies entspricht etwa der Trimerisierung von 40 bis 60 % der nach der Urethanisierung noch vorliegenden Isocyanatgruppen.

Der Abbruch der Trimerisierungsreaktion erfolgt durch Zugabe eines Katalysatorengifts, wobei als Katalysatorengifte beispielsweise Schwefel (im Falle der Verwendung von Phosphinen als Katalysatoren), sauer reagierende Substanzen (im Falle der Verwendung von Alkalisalzen, Alkalialkoholaten oder tert. Aminen als Katalysatoren) oder Alkylierungsmittel wie beispielsweise Toluolsulfonsäuremethylester (bei der bevorzugten Verwendung von Mannich-Basen als Katalysatoren) Verwendung finden können.

Die beim erfindungsgemäßen Verfahren als Verfahrensprodukte anfallenden Lösungen von Isocyanuratgruppen aufweisenden Polyisocyanaten weisen einen NCO-Gehalt von 8 bis 17 Gew.-%, bezogen auf Feststoff, auf. Die Lösungen zeichnen sich insbesondere durch eine verbesserte Verdünnbarkeit mit wenig polaren Lösungsmitteln der bereits obengenannten Art, insbesondere eine verbesserte Verdünnbarkeit mit aromatischen Lösungsmitteln wie Toluol oder Xylol aus. Im übrigen sind die Lösungen auch besser verträglich mit den üblichen, Hydroxylgruppen aufweisenden Reaktionspartnern, insbesondere mit fettsäuremodifizierten Polyestern, Hydroxylgruppen aufweisenden Polyacrylatharzen oder mit Hydroxylgruppen aufweisenden Zelluloseacetobutyraten.

In den nachfolgenden Beispielen beziehen sich alle Prozentangaben auf Gewichtsprozente.

In den nachfolgenden Beispielen wird als Katalysatorlösung eine 40-gew.-%ige Lösung in Xylol einer Mannich-Base der Formel

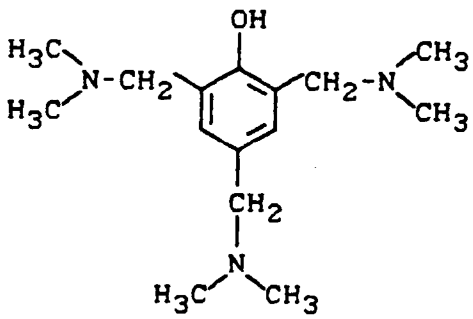

verwendet.

<u>Beispiel 1</u> (erfindungsgemäß)

375,5 g eines technischen Gemisches von 2,4- und 2,6-Diisocyanatotoluol im Verhältnis 4:1 werden bei 23°C mit 19,8 g 2-Ethylhexanol vermischt und unter Rühren vier Stunden auf 50°C erhitzt. Der NCO-Gehalt der Flüssigkeit fällt dabei auf 44,1 % ab. 3,5 % der vorhandenen NCO-Gruppen werden verbraucht. Man löst danach in 380 g Butylacetat und fügt dann 0,75 g der Katalysatorlösung zu. Die Trimerisierungsreaktion setzt sofort ein, die Temperatur der Lösung wird durch Kühlung auf 75°C gehalten. Nach jeweils 3 bis 4 Stunden werden insgesamt 3 mal weitere 0,75 g Katalysatorlösung zugefügt. Nach etwa 30 Stunden ist die Reaktion beendet. Zur Vernichtung des Katalysators fügt man 1 g Toluolsulfonsäuremethylester zu und erhitzt 1 Stunde auf 100°C. Man erhält eine klare, farblose Lösung mit folgenden Daten:
Konzentration: ca. 50 %
Viskosität: 1000 mPas/23°C
NCO-Gehalt: 7,8 %
freies Diisocyanat: 0,25 %

<u>Beispiel 2</u> (erfindungsgemäß)

Man verfährt wie in Beispiel 1, setzt aber als Monoalkohol 19,8 g 1-Dodecanol ein.
Man erhält eine Lösung eines Isocyanurat-Polyisocyanats mit folgenden Daten:
Konzentration: ca. 50 %
Viskosität: 800 mPas/23°C
NCO-Gehalt: 7,9 %
freies Diisocyanat: 0,1 %

<u>Beispiel 3</u> (erfindungsgemäß)

Man verfährt wie in Beispiel 1, setzt aber anstelle von 19,8 g Ethylhexanol 28,3 g 1-Dodecanol ein und erhöht die Butylacetatmenge um 8,5 g.
Man erhält eine Lösung eines Isocyanurat-Polyisocyanats mit folgenden Daten:
Konzentration: ca. 50 %
Viskosität: 1100 mPas/23°C
NCO-Gehalt: 7,7 %
freies Diisocyanat: 0,2 %

<u>Beispiel 4</u> (Vergleichsversuch)

Man verfährt wie in Beispiel 1, setzt aber als Alkohol 4,8 g Methanol zu und reduziert das Lösemittel, damit man wieder eine 50 %ige Lösung erhält.
Daten der Lösung:
Konzentration: ca. 50 %ig
Viskosität: 1100 mPas/23°C
NCO-Gehalt: 7,8 %
freies Diisocyanat: 0,28 %

Beispiel 5 (Vergleichsversuch)

Man verfährt wie in Beispiel 1, setzt aber 19,8 g Methanol ein.
Daten der Lösung:
Konzentration: ca. 50 %ig
Viskosität: 1800 mPas/23°C
NCO-Gehalt: 6,7 %
freies Diisocyanat: 0,2 %

Die zunächst klare Lösung wird beim Stehen nach etwa 8 Tagen trübe durch Abscheidung eines Niederschlages.

Beispiel 6 (Vergleichsversuch)

Man verfährt wie in Beispiel 1, verzichtet aber auf eine Modifizierung mit Alkohol und trimerisiert das Diisocyanat ohne Zusatz. Außerdem stellt man die Lösung mit Butylacetat auf 50 % ein.
Daten:
Konzentration: ca. 50 %
Viskosität: 1500 mPas/23°C
NCO-Gehalt: 7,9 %
freies Diisocyanat: 0,1 %

Beispiel 7 (Vergleichsversuch entsprechend DE-OS 2 414 413)

Man verfährt wie in Beispiel 6, gibt aber zu der erhaltenen Polyisocyanatlösung 19,8 g 2-Ethylhexanol und urethanisiert 4 Stunden bei 50°C. Man erhält eine klare Lösung.
Daten:
Konzentration: ca. 50 %ig
Viskosität: 2800 mPas/23°C
NCO-Gehalt: 6,8 %
freies Diisocyanat: 0,02 %

Die Lösung dieses Polyisocyanats hat vorteilhaft einen sehr niedrigen Gehalt an freiem Diisocyanat, aber eine ungünstig hohe Viskosität. Auch der niedrige NCO-Gehalt ist nachteilig.

Beispiel 8

Messung der Toluolverträglichkeit

Versuchsdurchführung: 15 - 20 g Polyisocyanatlösung werden genau eingewogen und unter Rühren mit Toluol versetzt (titriert). Die Menge Toluol wird angegeben, die die gleiche Trübung verursacht wie eine Standardlösung (0,4 g Büchsenmilch mit 10 % Fettgehalt in 100 g Wasser).

Die Ergebnisse sind in der Tabelle zusammengefaßt. Als Maßzahl für die Verdünnbarkeit dient das Verhältnis von Toluolverbrauch zur eingesetzten Isocyanatmenge.

**Tabelle**

| Polyisocyanatlösungen aus Beispiel | Einwaage g | Verbrauch ml Toluol | ml Toluol / g PIC |
|---|---|---|---|
| 1 | 16,381 | 40,46 | 2,47 |
| 2 | 17,941 | 53,39 | 2,53 |
| 3 | 16,735 | 48,69 | 2,91 |
| 4 | 15,963 | 19,95 | 1,25 |
| 5 | 17,452 | 20,07 | 1,15 |
| 6 | 18,725 | 23,03 | 1,23 |
| 7 | 19,223 | 25,95 | 1,35 |

Die Ergebnisse zeigen, daß die geforderte Eigenschaft, nämlich Verdünnbarkeit bzw. Verträglichkeit mit Toluol in optimaler Weise nur mit den erfindungsgemäßen Produkten erreicht wird. Entsprechend verbessert ist auch die Verträglichkeit mit anderen Bindemittelkomponenten und Lackharzen.

**Patentansprüche**

1. Verfahren zur Herstellung von Lösungen von Isocyanuratgruppen aufweisenden Polyisocyanaten in gegenüber Isocyanatgruppen inerten Lacklösungsmitteln durch Umsetzung von 2,5 bis 7 % der Isocyanatgruppen von 2,4-Diisocyanatotoluol oder von technischen Gemischen aus 2,4-Diisocyanatotoluol mit 2,6-Diisocyanatotoluol mit einem einwertigen Alkohol bei 0° bis 120°C und anschließende Trimerisierung eines Teils der nach der Urethanisierung noch vorliegenden Isocyanatgruppen in Gegenwart eines die Trimerisierung von Isocyanatgruppen beschleunigenden Katalysators bei 20° bis 80°C und Abbruch der Trimerisierungsreaktion durch Zugabe eines Katalysatorengifts, dadurch gekennzeichnet, daß man
   i) als einwertige Alkohole solche der allgemeinen Formel

$$R^1-[-O-CH_2-\underset{\displaystyle R^2}{\underset{|}{CH}}-]_n-OH$$

   verwendet, wobei
   $R^1$ für einen gegebenenfalls olefinisch ungesättigten (cyclo)aliphatischen Kohlenwasserstoffrest mit 6 bis 18 Kohlenstoffatomen steht,
   $R^2$ für Wasserstoff oder eine Methylgruppe steht und
   n für 0 oder eine ganze Zahl von 1 bis 3 steht,
   ii) die urethanisierten Diisocyanate der Trimerisierungsreaktion in Form von 30- bis 70-gew.-%igen Lösungen in gegenüber Isocyanatgruppen inerten Lacklösungsmitteln zuführt und
   iii) die Trimerisierungsreaktion durch Zugabe eines Katalysatorengifts abbricht, nachdem der Gehalt des Reaktionsgemischs an freiem Diisocyanatotoluol unter 0,5 Gew.-% liegt.

2. Verfahren gemäß Anspruch 1, dadurch gekennzeichnet, daß man als einwertige Alkohole Ethergruppen-freie Alkanole mit primären Hydroxylgruppen und 8 bis 12 Kohlenstoffatomen im Alkylrest verwendet.

3. Verfahren gemäß Anspruch 1 und 2, dadurch gekennzeichnet, daß man als einwertigen Alkohol 2-Ethylhexanol verwendet.

4. Verfahren gemäß Anspruch 1 bis 3, dadurch gekennzeichnet, daß man als Ausgangsdiisocyanat Gemische von 2,4-Diisocyanatotoluol mit 2,6-Diisocyanatotoluol im Gewichtsverhältnis 3:2 bis 9:1 verwendet.

5. Verwendung der gemäß Anspruch 1 bis 4 erhaltenen Lösungen von Isocyanuratgruppen aufweisenden Polyisocyanaten zur Herstellung von Zweikomponenten-Polyurethanlacken.

## Claims

1. A process for the production of solutions of isocyanurate polyisocyanates in paint solvents inert to isocyanate groups by reaction of 2.5 to 7% of the isocyanate groups of 2,4-diisocyanatotoluene or technical mixtures of 2,4-diisocyanatotoluene with 2,6-diisocyanatotoluene with a monohydric alcohol at 0 to 120°C and subsequent partial trimerization of the isocyanate groups still present in the presence of a catalyst which accelerates the trimerization of isocyanate groups at 20 to 80°C and termination of the trimerization reaction by addition of a catalyst poison, characterized in that
   i) the monohydric alcohols used correspond to the following general formula

$$R^1-[-O-CH_2-\underset{\displaystyle R^2}{\underset{|}{CH}}-]_n-OH$$

   in which
   $R^1$ is an optionally olefinically unsaturated (cyclo)aliphatic $C_{6-18}$ hydrocarbon radical,
   $R^2$ is hydrogen or a methyl group and
   n is 0 or an integer of 1 to 3,
   ii) the urethanized diisocyanates are subjected to the partial trimerization reaction in the form of 30 to 70% by weight solutions in paint solvents inert to isocyanate groups and
   iii) the trimerization reaction is terminated by addition of a catalyst poison after the content of free diisocyanatotoluene in the reaction mixture has fallen below 0.5% by weight.

2. A process as claimed in claim 1, characterized in that alkanols free from ether groups and containing primary hydroxyl groups and 8 to 12 carbon atoms in the alkyl radical are used as the monohydric alcohols.

3. A process as claimed in claims 1 and 2, characterized in that 2-ethyl hexanol is used as the monohydric alcohol.

4. A process as claimed in claims 1 to 3, characterized in that mixtures of 2,4-diisocyanatotoluene with 2,6-diisocyanatotoluene in a ratio by weight of 3:2 to 9:1 are used as the starting diisocyanate.

5. The use of the solutions of isocyanurate polyisocyanates obtained by the process claimed in claims 1 to 4 for the production of two-component polyurethane paints.

## Revendications

1. Procédé de préparation de solutions de polyisocyanates porteur de groupes isocyanurate dans des solvants pour peintures inertes vis-à-vis de groupes isocyanate, par réaction de 2,5 à 7 % des groupes isocyanate du 2,4-diisocyanatotoluène ou de mélanges techniques de 2,4-diisocyanatotoluène et de 2,6-diisocyanatotoluène avec un alcool monovalent à 0-120°C puis trimérisation d'une partie des groupes isocyanate encore présents après la formation d'uréthanne en présence d'un catalyseur accélérant la trimérisation de groupes isocyanate, à 20-80°C, et avec arrêt de la réaction de trimérisation par l'addition d'un poison pour le catalyseur, caractérisé en ce que :
   i) on utilise comme alcools monovalents, des alcools de formule générale

$$R^1-[-O-CH_2-\underset{\displaystyle R^2}{\underset{|}{CH}}-]_n-OH$$

dans laquelle

$R^1$ représente un reste hydrocarboné (cyclo) aliphatique présentant éventuellement une non-saturation oléfinique et ayant 6 à 18 atomes de carbone,

$R^2$ représente l'hydrogène ou un groupe méthyle et

n est égal à zéro ou à un nombre entier de 1 à 3,

ii) on fait passer les diisocyanates transformés en uréthanne de la réaction de trimérisation sous forme de solutions à 30-70 % en poids dans des solvants pour peintures inertes vis-à-vis de groupes isocyanate et

iii) on interrompt la réaction de trimérisation par addition d'un poison, pour le catalyseur, après que la teneur en diisocyanatotoluène libre du mélange réactionnel a atteint une valeur inférieure à 0,5 % en poids.

2. Procédé suivant la revendication 1, caractérisé en ce qu'on utilise comme alcools monovalents des alcanols dépourvus de groupes éther, portant des groupes hydroxyle primaires et ayant 8 à 12 atomes de carbone dans le reste alkyle.

3. Procédé suivant les revendications 1 et 2, caractérisé en ce qu'on utilise le 2-éthylhexanol comme alcool monovalent.

4. Procédé suivant les revendications 1 à 3, caractérisé en ce qu'on utilise comme diisocyanate de départ des mélanges de 2,4-diisocyanatotoluène et de 2,6-diisocyanatotoluène dans un rapport en poids de 3:2 à 9:1.

5. Utilisation des solutions, obtenues conformément aux revendications 1 à 4, de polyisocyanates porteurs de groupes isocyanurate pour la préparation de vernis de polyuréthanne à deux composants.